# EUROPEAN PATENT SPECIFICATION

(11) **EP 4 346 678 B1**
(45) Date of publication and mention of the grant of the patent: **29.04.2026**
(21) Application number: 22750743.1
(22) Date of filing: 18.05.2022
(51) Int. Cl.: A61B 34/20

(54) **MULTI-SENSOR ERROR CORRECTION FOR NAVIGATION**
MULTISENSORFEHLERKORREKTUR ZUR NAVIGATION
CORRECTION D'ERREUR À CAPTEURS MULTIPLES POUR NAVIGATION

(30) Priority: 27.05.2021 US 202163194038 P
(43) Date of publication of application: 10.04.2024
(73) Proprietor: Medtronic Navigation, Inc., Louisville, CO 80027 (US)
(72) Inventor: DOERR, Vince, Louisville, Colorado 80027 (US); SNYDER, Victor, Louisville, Colorado 80027 (US); JACOBSEN, Bradley, Louisville, Colorado 80027 (US)
(74) Representative: Viering, Jentschura & Partner mbB Patent- und Rechtsanwälte
(86) International application number: PCT/US2022/029766
(87) International publication number: WO 2022/251012

(56) References cited:
- WO-A1-2019/241655
- WO-A1-2021/003275
- US-B1- 10 539 644

## Description

### FIELD

The present technology generally relates to guided surgery and relates more particularly to electromagnetic surgical navigation.

### BACKGROUND

Electromagnetic surgical navigation may use an electromagnetic field and electromagnetic sensors to facilitate navigation of surgical tools in carrying out a surgical procedure. Electromagnetic sensors may provide data that may be used by the navigation system to localize a surgical tool relative to patient anatomy. Patient anatomy can change over time, particularly following placement of a medical implant or tracking device in or on patient anatomy. The presence of metal within the electromagnetic field may cause distortions that can lead to inaccurate measurements in field strength or in determining the positions of devices within the electromagnetic field.

A system according to the preamble of claim 1 is, e.g., known from WO 2019 / 241 655 A1.

US 10 539 644 B1 discloses an electromagnetic tracking (EMT) system which includes a tracked device, a tracking device, and a computing device. The EMT system is configured to receive, at the tracking device, an electromagnetic signal generated by the tracked device, determine, based on the electromagnetic signal, a set of possible positions and orientations of the tracked device relative to the tracking device, receive a measured inertial value representing a motion of the tracked device, determine an estimated inertial value corresponding to the motion of the tracked device based on at least one position and orientation of the set of possible positions and orientations, determine a difference value representing a difference between the estimated inertial value and the measured inertial value, determine a particular position and a particular orientation from the set in response to determining the difference value, and generate an output including the particular position and particular orientation.

WO 2021 / 003 275 A1 discloses a system for performing a surgical procedure on a bone of a patient. The system comprises a robotic arm including a proximal mounting portion, a distal tool interface for securing a surgical tool, and a plurality of joints between the proximal mounting portion and the distal tool interface for moving the distal tool interface in six degrees of freedom with respect to the proximal mounting portion. The system further comprises a j oystick configured to receive movement instructions for moving the distal tool interface in the six degrees of freedom and a processor configured to receive the movement instructions from the joystick; adjust the movement instructions based on one or more of a surgical plan and user input to generate adjusted movement instructions; and cause one or more of the plurality of joints to move the distal tool interface based on the adjusted movement instructions.

### SUMMARY

The invention provides a system according to claim 1. Further embodiments are described in the dependent claims. Any method and/or surgical step/procedure described in the present description is not covered by the invention as claimed, but is an example helpful for understanding the invention.

It is to be appreciated that any feature described herein can be claimed in combination with any other feature(s) as described herein, regardless of whether the features come from the same described embodiment.

The details of one or more aspects of the disclosure are set forth in the accompanying drawings and the description below. Other features, objects, and advantages of the techniques described in this disclosure will be apparent from the description and drawings, and from the claims.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X₁-Xₙ, Y₁-Yₘ, and Z₁-Zₒ, the phrase is intended to refer to a single element selected from X, Y, and Z, a combination of elements selected from the same class (e.g., X₁ and X₂) as well as a combination of elements selected from two or more classes (e.g., Y₁ and Zₒ).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

Numerous additional features and advantages of the present invention will become apparent to those skilled in the art upon consideration of the embodiment descriptions provided hereinbelow.

### BRIEF DESCRIPTION OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
Fig. 1 is a block diagram of a system according to at least one embodiment of the present disclosure;
Fig. 2A is a block diagram of components of the system according to at least one embodiment of the present disclosure;
Fig. 2B is a block diagram of components of the system according to at least one embodiment of the present disclosure;
Fig. 2C is a block diagram of components of the system according to at least one embodiment of the present disclosure;
Fig. 3 is a flowchart of a method to determine an accuracy of a first pose according to at least one embodiment of the present disclosure; and
Fig. 4 is a flowchart of a method according to at least one embodiment of the present disclosure.

### DETAILED DESCRIPTION

It should be understood that various aspects disclosed herein may be combined in different combinations than the combinations specifically presented in the description and accompanying drawings. It should also be understood that, depending on the example or embodiment, certain acts or events of any of the processes or methods described herein may be performed in a different sequence, and/or may be added, merged, or left out altogether (e.g., all described acts or events may not be necessary to carry out the disclosed techniques according to different embodiments of the present disclosure). In addition, while certain aspects of this disclosure are described as being performed by a single module or unit for purposes of clarity, it should be understood that the techniques of this disclosure may be performed by a combination of units or modules associated with, for example, a computing device and/or a medical device.

In one or more examples, the described methods, processes, and techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors (e.g., Intel Core i3, i5, i7, or i9 processors; Intel Celeron processors; Intel Xeon processors; Intel Pentium processors; AMD Ryzen processors; AMD Athlon processors; AMD Phenom processors; Apple A10 or 10X Fusion processors; Apple A11, A12, A12X, A12Z, or A13 Bionic processors; or any other general purpose microprocessors), graphics processing units (e.g., Nvidia GeForce RTX 2000-series processors, Nvidia GeForce RTX 3000-series processors, AMD Radeon RX 5000-series processors, AMD Radeon RX 6000-series processors, or any other graphics processing units), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Before any embodiments of the disclosure are explained in detail, it is to be understood that the disclosure is not limited in its application to the details of construction and the arrangement of components set forth in the following description or illustrated in the drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

The terms proximal and distal are used in this disclosure with their conventional medical meanings, proximal being closer to the operator or user of the system, and further from the region of surgical interest in or on the patient, and distal being closer to the region of surgical interest in or on the patient, and further from the operator or user of the system.

Electromagnetic navigation systems (e g., AxiEM^{™}Electromagnetic Technology) may have frustrated utility in the presence of metal. Metal in the navigation field may cause distortions, which can lead to errors in the calculated position and orientation. In particular, orientation errors can contribute to navigation errors. According to at least one embodiment of the present disclosure, a system is proposed which may use an inertial sensor (e.g., a sensor that uses a microelectromechanical systems (MEMS) sensor, multiple MEMS sensors, or non-MEMS sensors to measure inertial changes in position and/or orientation) as a redundant error correction device to correct for errors in orientation and/or position. The use of the MEMS sensor may be used by a navigation system to continue accurate navigation in the presence of metal, as discussed herein. It should be noted that while some embodiments discussed herein reference the use of MEMS sensors, it is to be understood that additional or alternative sensors (e.g., non-MEMS sensors) may be implemented. Any known or future sensor capable of determining or measuring pose or pose components may be implemented with the embodiments of the present disclosure.

Metal distortions may present challenges in ear, nose, and throat (ENT) and cranial surgery or surgical procedures. Embodiments of the present disclosure beneficially streamline procedures, reduce operating room time and costs, and improve navigation technology for semi-autonomous and fully autonomous surgeries by improving navigation accuracy in the presence of metal. Additionally, the presence of metal distortions may limit or prevent the use of electromagnetic (EM) navigation in robotic and non-robotic surgeries and/or procedures (such as spinal surgeries) due to the need to use instruments containing metal for completion of a surgery or surgical task. According to at least one embodiment of the present disclosure, using multiple sensor technologies to detect and correct errors that occur in one or more sensor types beneficially improves accuracy and allows for continued navigation without requiring the surgery or surgical task from being halted to address the error.

Embodiments of the present disclosure provide technical solutions to one or more of the problems of (1)pose errors arising from distances greater than the usable or functionable range of one or more sensors, and/or (2) pose errors arising from the presence of metal in an EM field during a surgery or surgical procedure.

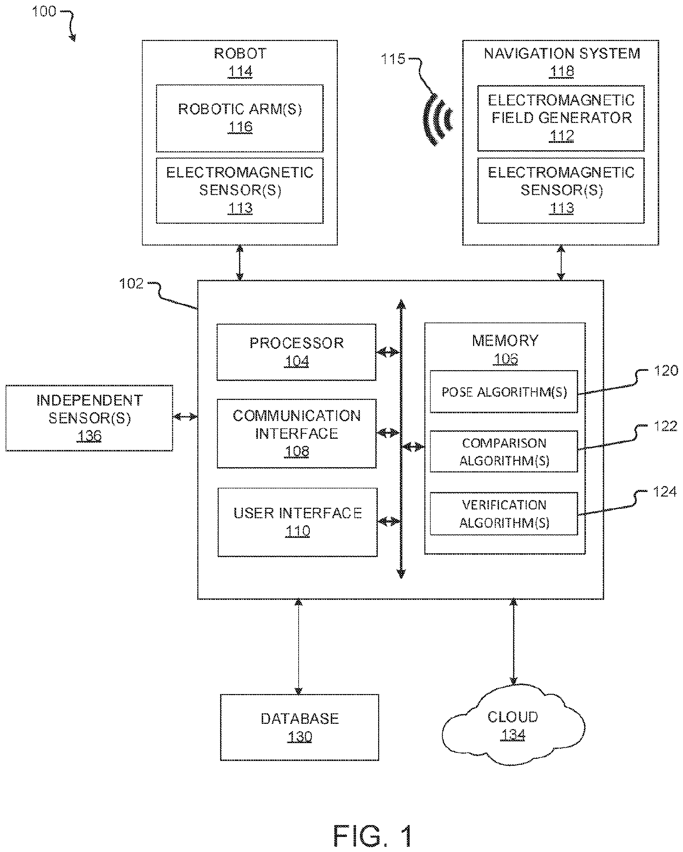

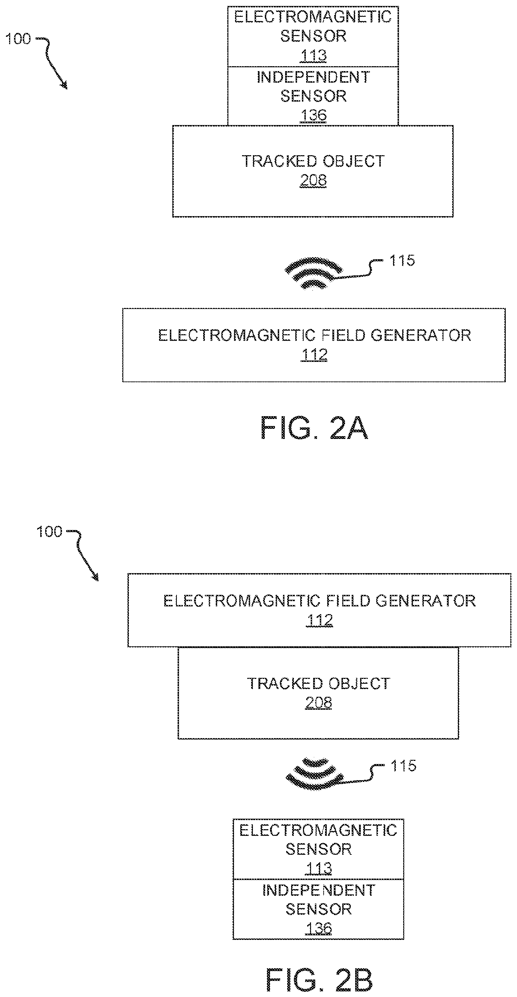

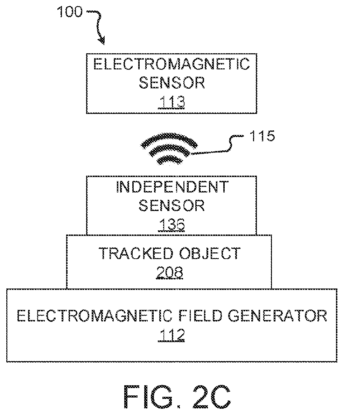

Turning first to Figs. 1 and 2, aspects of a system 100 according to at least one embodiment of the present disclosure is shown. The system 100 may be used to facilitate surgical navigation in an electromagnetic field; to determine pose and pose accuracy of one or more tracked objects (e.g., surgical tools, anatomical elements, robotic arms and/or end effectors, etc. within the electromagnetic field); to measure a change in pose of one or more surgical tools in the electromagnetic field based on sensor measurement; and/or carry out one or more other aspects of one or more of the methods disclosed herein. The system 100 comprises a computing device 102, an EM field generator 112, a robot 114, a navigation system 118, a database 130, and/or a cloud or other network 134. Systems according to other embodiments of the present disclosure may comprise more or fewer components than the system 100. For example, the system 100 may not include the robot 114, one or more components of the computing device 102, the database 130, and/or the cloud 134.

The computing device 102 comprises a processor 104, a memory 106, a communication interface 108, and a user interface 110. Computing devices according to other embodiments of the present disclosure may comprise more or fewer components than the computing device 102.

The processor 104 of the computing device 102 may be any processor described herein or any similar processor. The processor 104 may be configured to execute instructions stored in the memory 106, which instructions may cause the processor 104 to carry out one or more computing steps utilizing or based on data received from the robot 114, the navigation system 118, the database 130, and/or the cloud 134.

The memory 106 may be or comprise RAM, DRAM, SDRAM, flash memory, other solid-state memory, any memory described herein, or any other tangible, non-transitory memory for storing computer-readable data and/or instructions. The memory 106 may store information or data useful for completing, for example, any step of the methods 300 and/or 400 described herein, or of any other methods. The memory 106 may store, for example, one or more pose algorithms 120, one or more comparison algorithms 122, and/or one or more verification algorithms 124. Such instructions or algorithms may, in some embodiments, be organized into one or more applications, modules, packages, layers, or engines. The algorithms and/or instructions may cause the processor 104 to manipulate data stored in the memory 106 and/or received from or via the robot 114, the database 130, and/or the cloud 134. Alternatively or additionally, functionality provided by the various components of memory 106 depicted and described herein can be provided by a neural network, artificial neural network, or other type of machine learning model. Thus, while various components of memory 106 are depicted as instructions, it should be appreciated that some or all of these components may be provided as an artificial neural network and may provide similar functionality as the instructions described herein.

The computing device 102 may also comprise a communication interface 108. The communication interface 108 may be used for receiving image data or other information from an external source (such as the robot 114, the navigation system 118, the database 130, the cloud 134, and/or any other system or component not part of the system 100), and/or for transmitting instructions, images, or other information to an external system or device (e.g., another computing device 102, the robot 114, the navigation system 118, the database 130, the cloud 134, and/or any other system or component not part of the system 100). The communication interface 108 may comprise one or more wired interfaces (e.g., a USB port, an ethernet port, a Firewire port) and/or one or more wireless transceivers or interfaces (configured, for example, to transmit and/or receive information via one or more wireless communication protocols such as 802.11a/b/g/n, Bluetooth, NFC, ZigBee, and so forth). In some embodiments, the communication interface 108 may be useful for enabling the device 102 to communicate with one or more other processors 104 or computing devices 102, whether to reduce the time needed to accomplish a computing-intensive task or for any other reason.

The computing device 102 may also comprise one or more user interfaces 110. The user interface 110 may be or comprise a keyboard, mouse, trackball, monitor, television, screen, touchscreen, and/or any other device for receiving information from a user and/or for providing information to a user. The user interface 110 may be used, for example, to receive a user selection or other user input regarding any step of any method described herein. Notwithstanding the foregoing, any required input for any step of any method described herein may be generated automatically by the system 100 (e.g., by the processor 104 or another component of the system 100) or received by the system 100 from a source external to the system 100. In some embodiments, the user interface 110 may be useful to allow a surgeon or other user to modify instructions to be executed by the processor 104 according to one or more embodiments of the present disclosure, and/or to modify or adjust a setting of other information displayed on the user interface 110 or corresponding thereto.

Although the user interface 110 is shown as part of the computing device 102, in some embodiments, the computing device 102 may utilize a user interface 110 that is housed separately from one or more remaining components of the computing device 102. In some embodiments, the user interface 110 may be located proximate one or more other components of the computing device 102, while in other embodiments, the user interface 110 may be located remotely from one or more other components of the computer device 102.

The robot 114 may be any surgical robot or surgical robotic system. The robot 114 may be or comprise, for example, the Mazor X^{™} Stealth Edition robotic guidance system. The robot 114 may be configured to position a surgical tool at one or more precise position(s) and orientation(s), and/or to return the surgical tool to the same position(s) and orientation(s) at a later point in time. The robot 114 may additionally or alternatively be configured to manipulate a surgical tool (whether based on guidance from the navigation system 118 or not) to accomplish or to assist with a surgical task. In some embodiments, the robot 114 may be configured to hold and/or manipulate an anatomical element during or in connection with a surgical procedure. The robot 114 may comprise one or more robotic arms 116. In some embodiments, the robotic arm 116 may comprise a first robotic arm and a second robotic arm, though the robot 114 may comprise more than two robotic arms. In some embodiments, one or more of the robotic arms 116 may be used to hold and/or maneuver a surgical tool. Each robotic arm 116 may be positionable independently of the other robotic arm. The robotic arms may be controlled in a single, shared coordinate space, or in separate coordinate spaces.

The robot 114, together with the robotic arm 116, may have, for example, one, two, three, four, five, six, seven, or more degrees of freedom. Further, the robotic arm 116 may be positioned or positionable in any pose, plane, and/or focal point. The pose includes a position and an orientation. As a result, a surgical tool, or other object held by the robot 114 (or, more specifically, by the robotic arm 116) may be precisely positionable in one or more needed and specific positions and orientations.

The robotic arm(s) 116 may comprise one or more sensors that enable the processor 104 (or a processor of the robot 1 14) to determine a precise pose in space of the robotic arm (as well as any object or element held by or secured to the robotic arm).

The navigation system 118 may provide navigation for a surgeon and/or a surgical robot during an operation and may comprise an electromagnetic (EM) field generator 112 and an EM sensor 113. The navigation system 118 may be any now-known or future-developed navigation system, including, for example, the Medtronic StealthStation^{™} S8 surgical navigation system or any successor thereof. The navigation system 118 may include one or more cameras or other sensor(s) for tracking one or more reference markers, navigated trackers, or other objects within the operating room or other room in which some or all of the system 100 is located. The one or more cameras may be optical cameras, infrared cameras, or other cameras. In various embodiments, the navigation system 118 may be used to track a position and orientation (i.e., pose) of the robot 114 and/or robotic arm 116, and/or one or more surgical tools (or, more particularly, to track a pose of a navigated tracker and/or sensor attached, directly or indirectly, in fixed relation to the one or more of the foregoing) relative to, for example, an anatomical element (e.g., a vertebra). In some embodiments, the navigation system 118 may additionally or alternatively track one or more anatomical elements or other rigid anatomy (e.g., vertebrae, ribs, sternum, femurs, patellae, etc.) using one or more sensors (e.g., the independent sensor 136, the EM sensor 113, combinations thereof, and/or the like) in conjunction with the generated EM field 115. The navigation system 118 may include a display for displaying one or more images from an external source (e.g., the computing device 102 or other source) or for displaying an image and/or video stream from the one or more cameras or other sensors of the navigation system 118. In some embodiments, the system 100 can operate without the use of the navigation system 118. The navigation system 118 may be configured to provide guidance to a surgeon or other user of the system 100 or a component thereof, to the robot 114, or to any other element of the system 100 regarding, for example, a pose of one or more anatomical elements, whether or not a tool is in the proper trajectory, and/or how to move a tool into the proper trajectory to carry out a surgical task according to a preoperative or other surgical plan.

The EM field generator 112 is configured to generate one or more EM fields 115. The EM field 115 generated by the EM field generator 112 is used by the system 100 and/or one or more components thereof (e.g., the computing device 102) to determine the pose of one or more components of the system 100 within the EM field 115 (e.g., a pose of a surgical tool navigated through the EM field 115 during the course of a surgery or surgical procedure, a pose of a sensor device attached to the robot 114 and/or the robotic arm 116, etc.). In some embodiments, the EM field 115 may be localized. For example, the EM field 115 may be generated locally near, around, and/or in proximity to a patient, such that movement of metal outside the range of the EM field 115 (e.g., outside of an operating room) does not measurably impact the generation or measurability of the EM field 115 (or vice versa). In some embodiments, the EM field generator 112 may be positioned in proximity to a patient (e.g., underneath the patient on an operating table) and/or may be oriented such that the EM field 115 generated can be used to assist in navigation of one or more components of the system 100 (e.g., a surgical tool connected to a robot 114 or a robotic arm 116). For example, the navigation system 118 may use the EM field 115 in conjunction with the one or more EM sensors 113 to determine the pose of surgical tools, robotic arms, anatomical elements or objects, combinations thereof, and/or the like within the EM field 115.

The one or more EM sensors 113 are configured to measure an EM field (e.g., the EM field 115 generated by the EM field generator 112) and/or changes thereto. For example, the EM sensor 113 may comprise one or more metal coils (e.g., three independent metal coils orthogonally aligned) configured to measure components of the EM field 115 and output the measurements (e.g., to a computing device 102) as an electronic signal. In this way, the EM sensor 113 may be considered a transducer that converts a sensed EM field, such as the EM field 115, into an electrical signal or output. The EM sensor 113 may generate one or more data sets comprising measurements related to the EM field 115 and/or changes in the EM field 115. The one or more datasets may be processed (e.g., by an algorithm such as the pose algorithm 120) to determine a pose of one or more components of the system 100 (e.g., the robot 114, the robotic arm 116 and/or a surgical tool attached thereto, an anatomical element or object, etc.) within the EM field 115.

In some embodiments, when the EM field 115 as measured by the EM sensor 113 changes (e.g., due to the presence and/or movement of metal or other objects in the EM field 115), the change may create voltage across the metal coils of the EM sensor 113. The generated voltage may be measured and relayed to one or more components of the system 100 (e.g., the computing device 102, database 130, etc.) to determine the relative strength and orientation of the EM field 115 at various locations and/or to determine a change in pose of one or more components of the system 100 (e.g., a surgical tool) within the EM field 115. Additionally or alternatively, different parameters may be captured and/or measured (e.g., voltage, current, resistance, inductance, capacitance, combinations thereof, and the like) and relayed to the system 100 (and/or one or more components thereof such as the computing device 102) to determine the strength, direction, orientation, and/or change in the EM field 115 and/or other objects within the EM field 115 (e.g., elements containing conductive materials such as a surgical tool, anatomical elements, or objects to which the EM sensor 113 is attached, etc.).

In some embodiments, the EM sensor 113 may be configured to measure the EM field 115 in multiple directions or may measure the EM field 115 using multiple unidirectional sensors. The EM sensor 113 may, for example, measure each vector component of the EM field 115 (e.g., in an X-axis direction, a Y-axis direction, Z-axis direction, etc.) as the EM sensor 113 changes relative to the EM field 115 (e.g., as the EM sensor 113 moves through the EM field 115 on a surgical tool or anatomical element).

In some embodiments, the EM sensor 113 and/or the independent sensor 136 may generate measurements that are used by the system 100 to determine or correct poses associated with tracked objects (e.g., surgical tools, anatomical elements, etc.) when the EM sensor 113 is at a greater distance than the usable or functionable range of the EM sensor 113, and/or when the EM sensor 113 cannot properly measure the EM field 115 (possible due to the EM field generator 112 failing to generate a strong enough field at the distance). For instance, the EM sensor 113 may be on a tracked object that is positioned on the border of the effective range of the EM sensor 1 13 such that the EM sensor 113 provides inaccurate measurements of the EM field 115. Additionally or alternatively, the EM field 115 may be disrupted by noise (e.g., distortions or fluctuations in the magnitude and/or frequency of the EM field 115) which may subsequently exacerbate or amplify the inaccuracy of measurements made by the EM sensor 113, and/or may be too weak for the EM sensor 113 to accurately detect the EM field 115 at the location of the tracked object.

In such embodiments, the independent sensor 136 may be used to as a supplement to or in lieu of the measurements generated by the EM sensor 113 to determine an accurate pose of the tracked object.

With continued reference to Figs. 2A-2C, block diagrams of components of the system 100 according to at least one embodiment of the present disclosure is shown. The components include an EM field generator 112, an EM sensor 113, an independent sensor 136, and a tracked object 208. Notwithstanding the foregoing, systems according to other embodiments of the present disclosure may omit one or more aspects of the system 100 illustrated in Figs. 2A-2C, such as the independent sensor 136. Additionally, systems according to other embodiments of the present disclosure may arrange one or more components of the system 100 differently (e.g., the EM field generator 112, the EM sensor 113, and/or the independent sensor 136 may comprise one or more components of the computing device 102, and/or vice versa), and/or may include additional components not depicted in Figs. 2A-2C.

The independent sensor 136 is configured to measure one or more movements in position or rotation and produce data associated therewith. For instance, the independent sensor 136 may be or comprise an inertial sensor (e.g., an accelerometer, a gyroscope, a microelectromechanical sensor (MEMS), combinations thereof, etc.) that measures an acceleration, rotation, or other movement of the sensor by tracking, for example, one or more vectors, such as acceleration and/or force vectors (e.g., a gravity vector). The independent sensor 136outputs the measurements to the system 100 and/or one or more components thereof (e.g., the computing device 102), which may be useful in determining the pose of the independent sensor 136and/or components attached thereto (e.g., a surgical tool, the robot 114 and/or the robotic arm 116, combinations thereof, and/or the like).

In some embodiments, the independent sensor 136 may be positioned a first distance relative to a surgical tool. For example, the independent sensor 136 may be attached to the robotic arm 116 holding the surgical tool. The independent sensor 136 on the robotic arm 116 may measure the relative movement of the robotic arm 116 and provide the movement measurements to the computing device 102 or the processor 104, which may use the measurements in one or more algorithms to determine the pose of the surgical tool. In some embodiments, the independent sensor 136 may be positioned on the surgical tool (i.e., the first distance between the independent sensor 136 and the surgical tool is fixed and may be near zero or completely zero).

In at least one embodiment, the independent sensor 136 is attached to the EM sensor 113 or vice versa. In this embodiment, the EM sensor 113 (or the independent sensor 136) may be attached to a surgical tool (e.g., a tool held by the robotic arm 116) or an anatomical element (e.g., a vertebra or a portion thereof), such that any movement of the anatomical element is detected by the independent sensor 136. In one example, the independent sensor 136 may be attached to the EM sensor 113 and the pair of sensors are attached to a vertebra for the purposes of carrying out a surgery or surgical procedure thereon (e.g., a drilling procedure). Any movement of the vertebra during the surgery or surgical procedure may be detected by the system 100 based on the measurements of the independent sensor 136 and/or the EM sensor 113. For example, a surgical tool may be in a first pose as determined by the system 100. During the course of the surgery or surgical procedure, the surgical tool may move, causing the vertebra to move as well. The independent sensor 136 and/or the EM sensor 113 may then output data sets indicating that the surgical tool has moved from the first pose. The data sets may then be used to determine the new pose of the surgical tool, the new pose of the vertebra, or combinations thereof. In some embodiments, such as when metal is introduced to the EM field, the surgical tool or the anatomical element may remain stationary (i.e., not moving) and the independent sensor 136 may provide a similar or the same measurement as before the metal entered the EM field (e.g., the independent sensor 136 measures an gravitational force in a first direction both before and after the metal has been introduced to the EM field), but the EM sensor 113 may measure a different pose of the surgical tool or the anatomical element based on the introduction of metal into the EM field (e.g., the EM sensor 113 initial measured a first orientation, but now says the orientation has changed without the surgical tool or the anatomical element having actually moved). In such embodiments, the system 100 and/or one or more components thereof (e.g., the computing device 102) may be able to identify the EM sensor 113 measurements as inaccurate and/or omit the use of the EM sensor 113 measurements.

In some embodiments, such as the embodiment depicted in Fig. 2B, the EM field generator 112 (rather than the EM sensor 113 and/or the independent sensor 136) may be attached to the tracked object 208. In such embodiments, the EM sensor 113 and/or the independent sensor 136 may be placed outside of the tracked object 208. While the EM sensor 113 may measure changes in the EM field 115, the independent sensor 136 may be attached to the EM sensor 113 and may measure inertial changes in the EM sensor 113. Additionally or alternatively, combinations of the EM field generator 112, the EM sensor 113, and the independent sensor 136 may be attached to the tracked object 208. For example, as depicted in Fig. 2C, the EM field generator 112 and the independent sensor 136 may be attached to the tracked object 208 and the EM sensor 1 13 may be positioned outside of the tracked object 208.

It may also be possible to provide a single sensor device that provides functionality of the EM sensor 113 and the independent sensor 136. For example, a single sensor device may be configured to measure an EM field (e.g., the EM field 115) in addition to tracking a motion or position (absolute or relative) of the object to which the sensor is attached. Alternatively, a sensor system is contemplated that may include the EM sensor 113 and one or more independent sensors 136 in a common package (e.g., attached to a common substrate or contained within a common housing).

In some embodiments, the independent sensor 136 and/or the EM sensor 113 may be substantially smaller than the surgical tool (and/or other components of the system 100). Unless otherwise specified, "substantially smaller" as used herein means that the attachment of the independent sensor 136 and/or the EM sensor 113 to the component of the system 100 (e.g., the surgical tool) does not alter the form factor (i.e., the overall shape) thereof. The overall shape may be the outline or curvature created by the component of the system 100. Alternatively, when so specified, "substantially smaller" means that a volume occupied by the independent sensor 136 and/or the EM sensor 113 is less than a volume occupied by the component of the system 100 (e.g., less than 0.1%, 0.2%, 1%, 2%, 5%, etc.). For the avoidance of doubt, each of the foregoing definitions of "substantially smaller" can be used in connection with any embodiment described herein within the scope of the present disclosure.

The system 100 or similar systems may be used, for example, to carry out one or more aspects of any of the methods 300 and/or 400 described herein. The system 100 or similar systems may also be used for other purposes.

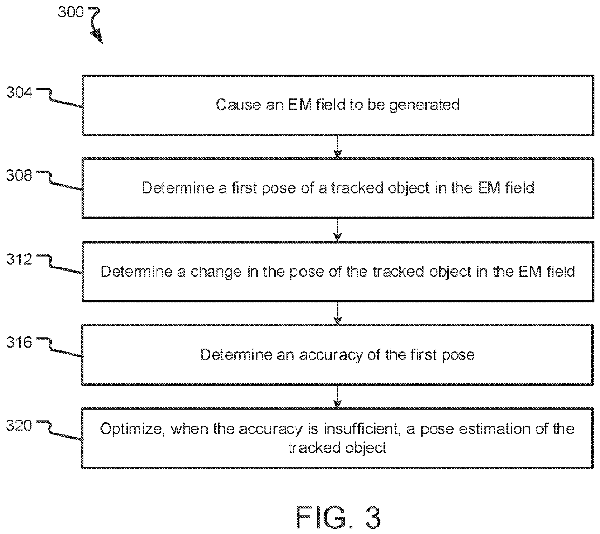

Fig. 3 depicts a method 300 that may be used, for example, to verify the accuracy of a pose of a surgical tool or anatomical element in an electromagnetic field using, for example, data received from an EM sensor and an independent sensor.

The method 300 (and/or one or more steps thereof) may be carried out or otherwise performed, for example, by at least one processor. The at least one processor may be the same as or similar to the processor(s) 104 of the computing device 102 described above. The at least one processor may be part of a robot (such as a robot 114) or part of a navigation system (such as a navigation system 118). A processor other than any processor described herein may also be used to execute the method 300. The at least one processor may perform the method 300 by executing instructions stored in a memory such as the memory 106. The instructions may correspond to one or more steps of the method 300 described below. The instructions may cause the processor to execute one or more algorithms, such as a pose algorithm 120, a comparison algorithm 122, and/or a verification algorithm 124.

The method 300 comprises causing an EM field to be generated (step 304). The EM field (e.g., an EM field 115) may be generated, for example, by an EM field generator (e.g., an EM field generator 112) or by the processor described above (e.g., a processor 104), which may provide an activation signal to the EM field generator 112. In some embodiments, the EM field may be produced constantly or periodically during the course of a surgery or surgical procedure. As previously described, the EM field may be localized to a location or spatial volume around a patient, such that the EM field is more sensitive to conductive materials or the movement of metal near the patient, and less sensitive to metal or the movement of metal further away from the patient (e.g., outside an operating room).

The method 300 also comprises determining a first pose of a tracked object in the EM field (step 308). The tracked object may be, for example, a surgical tool, an anatomical element (e.g., a vertebra, a rib, etc.), a robot end effector, or another component of a robot or robotic arm. The first pose may be determined based on data sets generated by a sensor such as an independent sensor 136 and/or an EM sensor such as an EM sensor 113. For example, the EM sensor may be affixed to the tracked object 1, which may be positioned within the EM field generated in the step 304. The EM sensor may then measure the EM field (e.g., using three or more independent sensing coils) and/or changes thereto and generate one or more data sets reflecting the measurements of the EM field. In some embodiments, the EM sensor may be or comprise one or more magnetometers, such as conductive wirings wrapped around one or more iron cores (e.g., a fluxgate) or a superconducting quantum interference device (SQUID) magnetometer and/or may additionally or alternatively comprise one or more induction coils. The pose of the tracked object can then be determined based on the readings generated by the EM sensor. In some embodiments, the tracked object may be positioned, moved, or oriented such that the tracked object affects the EM field measurement at the EM sensor, which in turn affects the datasets output from the EM sensor. For example, the EM sensor may be attached to the tracked object, and the tracked object may contain metal (e.g., a surgical tool with metal portions) such that the measurements generated by the EM sensor may be distorted. In another example, the tracked object may be positioned such that the EM sensor measurement is affected by the pose of the tracked object (e.g., the tracked object blocks the EM sensor from reading one or more components of the EM field).

The one or more datasets generated by the EM sensor are passed to a pose algorithm (e.g., a pose algorithm 120) to determine the pose of the tracked object based on the one or more datasets. The pose algorithm takes the one or more datasets as an input, as well as additional EM field data (e.g., the initial strength and orientation of the EM field as determined by the EM generator) and may output a determined pose of the tracked object. The determined pose information may be stored (e.g., in a database 130), and/or may be sent to one or more components of a system (e.g., a system 100), such as a computing device 102.

The method 300 also comprises determining a change in the pose of the tracked object in the EM field (step 312). The change in pose may result from movement of the tracked object and/or from movement in one or more anatomical elements or objects (e.g., a vertebra) during a surgery or surgical procedure. For example, the tracked object may be a surgical tool that is caused to move from a first position to a second position in the EM field, such as when the surgical tool is being moved into position to drill into a vertebra. As such, changes in the pose of the surgical tool may impact the measurement of the EM field by the EM sensor. In some embodiments, the EM sensor may return different measurements of the EM field after the surgical tool has moved to the second position. The new measurements may be reflected in the datasets generated by the EM sensor, and the datasets may be input into a comparison algorithm (e.g., a comparison algorithm 122) along with the previous datasets comprising the original EM field measurements. The comparison algorithm may compare the new datasets to previous datasets (e.g., datasets related to the EM field measurements when the surgical tool was in the first position) to determine changes in the EM field. The comparison algorithm may then output a confirmation that the pose of the surgical tool has changed, which may be sent to one or more components of the system (e.g., a computing device 102, a database 130, etc.). After the system has confirmed that the pose of the tracked object has changed, the system may calculate the new pose of the tracked object in the EM field using the new EM field measurements generated by the EM sensor, which may use a similar or the same approach as the step 308.

In some embodiments, the change in pose may be based on a threshold value. The threshold value may indicate a degree of similarity between the new datasets and the previous datasets (and by extension, the degree of similarity of respective poses). For example, a change caused by a movement of the tracked object in a first direction may be reflected in a difference in one or more portions of the new dataset when evaluated by the comparison algorithm (e.g., portions of the new dataset may contain EM field data associated with the first direction that are different in value, sign, metadata, etc. from the data associated with the first direction in the previous dataset). The comparison algorithm may then indicate that the pose of the tracked object has changed if the difference between the datasets is above (or in some cases below) the threshold value. In some embodiments, the threshold value may be predetermined by the system, components thereof, and/or the surgeon. In some embodiments, the threshold value may indicate that a degree of difference is statistically significant, or otherwise significant to the surgery or surgical procedure. In such embodiments, for instance, falling below the threshold value may indicate that the readings from the EM sensor are sufficiently similar to treat the second position of the tracked object as the same as the first position, which may indicate that the change in pose was not significant enough to impact the surgery or surgical procedure.

The method 300 also comprises determining an accuracy of the first pose (step 316). The verifying may include instances where the tracked object (or other components of the system) contain metal and, when the tracked object moves, the metal may disrupt or otherwise change the measurements generated by the EM sensor even when the tracked object does not move. In such embodiments, the system may verify the accuracy of the new pose of the tracked object using measurements generated by the independent sensor (e.g., by an independent sensor 136). For instance, the sensor may be positioned proximate to or on the EM sensor and may generate measurements related to inertial readings or movements (e.g., positional changes and/or rotational changes) of one or more components of the system (e.g., the surgical tool). The system may receive the measurements from the independent sensor and determine whether a change in pose has occurred based on whether the independent sensor measures inertial movements that coincide with the changes in measurements from the EM sensor.

As an example, the tracked object may be a surgical tool that comprises metal that distorts the EM field (or measurements thereof), causing the measurements of the EM sensor to incorrectly reflect the change in pose (e.g., one or more measurements by the EM sensor show the EM field as larger, smaller, in a different direction, and/or with a different phase than the EM field generated by the EM field generator) which may result in an incorrect pose determination when the system uses the EM sensor measurements to determine the new pose. The surgical tool may be tracked (e.g., the system utilizes the measurements generated by the EM sensor to determine the pose of the surgical tool) or non-tracked (e.g., the EM sensor measurements are not used in determining the pose of the surgical tool). In another example, the tracked object (e.g., a surgical tool and/or an anatomical element) and the EM sensor may be stationary in the EM field, and another non-tracked object containing metal (e.g., another surgical tool, a robotic arm, etc.) enters the EM field. The metal of the non-tracked object may cause the measurements by the EM sensor to be distorted, resulting in system error in tracking the pose of the tracked object.

The system may verify the accuracy of the EM measurements by comparing the determined pose with a pose calculated based on the independent sensor. For instance, the system may use a verification algorithm (e.g., a verification algorithm 124) to determine the pose of the surgical tool using measurements supplied by the independent sensor. In at least one embodiment, the verification algorithm may receive the measurements from the independent sensor and determine a posed based on the initial pose of the surgical instrument and the measurements from the independent sensor (e.g., by calculating the change in pose of the surgical tool as measured by the independent sensor). The verification algorithm may then compare the independent sensor pose with the determined pose based on the measurements supplied by the EM sensor and determine whether the two poses match (or are within a sufficiently acceptable degree of difference). In some embodiments, when the two poses match (or are sufficiently close in pose based on, for example, a threshold value), the verification algorithm may indicate to the system (e.g., via an electronic message) that the new pose is accurate. In some embodiments, the system and/or one or more components thereof (e.g., the surgical tool) may be instructed (e.g., controlled by a processor) to await receipt of the indication that the new pose is accurate before continuing with the surgery or surgical procedure. The surgical tool may be locked, idled, or may otherwise fail to operate until the indication is received. In another example, the image guidance (e.g., a navigation system based on images or an image feed to maneuver robotic arms) may be disabled until the indication is received.

The method 300 also comprises optimizing, when the accuracy is insufficient, a positional estimation of the tracked object (step 320). The pose estimation of the new pose may be based on measurements generated by the sensor, the EM sensor, combinations thereof, and/or the like. For instance, in some embodiments the system may determine that the first pose is inaccurate (e.g., the accuracy falls below a threshold value, the accuracy is insufficient to continue the surgery or surgical procedure, etc.) and may estimate the new pose based on available data (e.g., historical measurements and/or current measurements generated by one or more sensors in the system).

In at least one embodiment, the processor may exclude or omit from use certain data sets that are deemed inaccurate by the system when estimating the new pose. For example, the independent sensor may provide a gravitational vector measurement in a first direction that does not change as the tracked object changes pose, while the EM sensor may provide a first measurement indicating a change in pose and a second measurement indicating that the pose has not changed. In this example, the processor may exclude the second measurement in the pose estimation, since the second measurement does not accurately reflect the pose change of the tracked object.

In some embodiments, the processor may use a pose algorithm (e.g., a pose algorithm 120) to determine the new pose of the tracked object. The pose algorithm may receive one or more datasets (e.g., generated from the independent sensor, the EM sensor, combinations thereof, and/or the like) and determine a pose based thereon. As previously noted, the processor may only pass measurements to the pose algorithm that are deemed accurate when estimating the new pose, and may omit inaccurate measurements.

The present disclosure encompasses embodiments of the method 300 that comprise more or fewer steps than those described above, and/or one or more steps that are different than the steps described above.

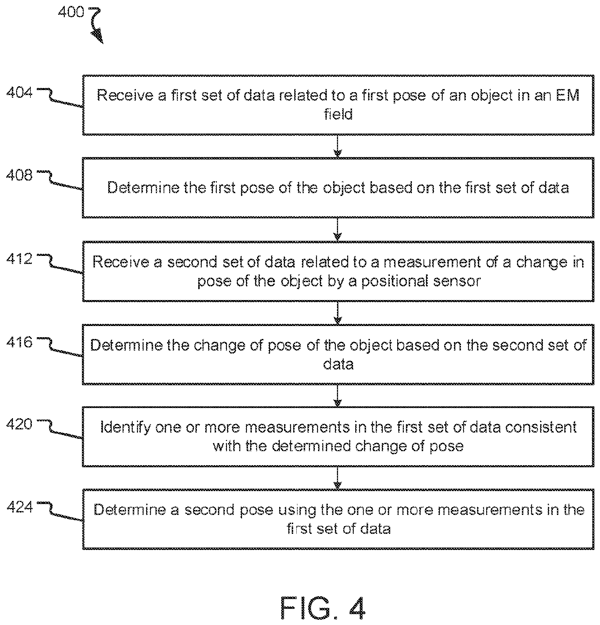

Fig. 4 depicts a method 400 that may be used, for example, to verify and correct navigation errors for a surgical tool in an EM field. The method 400, or one or more aspects thereof, may be used to, for example, identify a change in pose of a surgical tool within the EM field and correct the error by updating the registration, navigation, combinations thereof, and/or the like.

The method 400 (and/or one or more steps thereof) may be carried out or otherwise performed, for example, by at least one processor. The at least one processor may be the same as or similar to the processor(s) 104 of the computing device 102 described above. The at least one processor may be part of a robot (such as a robot 114) or part of a navigation system (such as a navigation system 118). A processor other than any processor described herein may also be used to execute the method 400. The at least one processor may perform the method 400 by executing instructions stored in a memory such as the memory 106. The instructions may correspond to one or more steps of the method 400 described below. The instructions may cause the processor to execute one or more algorithms, such as a pose algorithm 120, a comparison algorithm 122, and/or a verification algorithm 124.

The method 400 comprises receiving a set of data related to a first pose of an object in an EM field (step 404). The object may be a component of a system (e.g., a system 100), such as a surgical tool, an anatomical element (e.g., a vertebra), combinations thereof, and/or the like. The set of data is generated, for example, by an EM sensor (e.g., an EM sensor 113) related to one or more measurements of an EM field. In some embodiments, the set of data may comprise information related to multiple directions and/or orientations of the EM field. The set of data may be saved or stored in one or more components of a system (e.g., a computing device 102, a database 130, etc.).

The method 400 also comprises determining the first pose of the object based on the first set of data (step 408). The step 408 may be similar to or the same as the step 308 as described above in the method 300. For instance, the first pose may be determined using a pose algorithm (e.g., a pose algorithm 120) based on the first set of data received in the step 404.

The method 400 also comprises receiving a second set of data related to a measurement of a change in pose of the object by an independent sensor (step 412). The second set of data is generated by an independent sensor (e.g., an independent sensor 136). The second set of data contains information related to a measurement of a change in pose of one or more components of a system (e.g., a system 100) that is measured, for example, as the one or more components onto which the independent sensor is attached. For example, the independent sensor may be attached to an anatomical element (e.g., a vertebra) and, when the vertebra moves within the EM field, the independent sensor captures information related to the movement and transmits the data to one or more components of the system (e.g., to a computing device 102, to a database 130, etc.). In some embodiments, the second set of data may be or comprise information directed to inertial readings (e.g., accelerations, rotations, vibrations, etc.) produced by the anatomical element in the system. In such embodiments, the inertial readings may be measured by the independent sensor and stored in, for example, a database or other computing device.

In some embodiments, the independent sensor may be placed a first distance from the anatomical element (or other component in the system) and configured to capture measurements related to the movement of the anatomical element relative to the independent sensor. For example, the independent sensor may measure a one or more distances (e.g., 1 millimeter (inin), 2 mm, 5 mm, 10 mm, 1 meter (mm), 5m, 10 m, etc.) between the independent sensor and a reference point on the anatomical element (e.g., the closest point on the anatomical element to the independent sensor, the tip of a surgical tool performing a surgical procedure on the anatomical element, etc.), such that any change in pose may be reflected in the second set of data when compared with the first set of data (with the first set of data reflecting an initial pose of the anatomical element).

In at least one embodiment, the independent sensor may be attached to the EM sensor (or vice versa). In this embodiment, the independent sensor and the EM sensor may be attached to one or more components of a system (e.g., a system 100), such as a surgical tool or a vertebra (in instances where a surgery or surgical procedure is being performed on the vertebra). The attachment of both the independent sensor and the EM sensor may permit for measurements in both the EM field and any physical or inertial movement of the component to be generated from a common location, which may simplify determinations by the system of any pose changes of the components of the system to which the EM sensor and the independent sensor are attached.

The method 400 also comprises determining a change in pose of the object based on the second set of data (step 416). The second set of data is received from the independent sensor, which may be positioned on or near a surgical tool or anatomical element (e.g., a vertebra) and configured to detect changes in physical location or orientation of the component to which the independent sensor is attached. The system may use data from the independent sensor to determine a change in pose of the surgical tool or anatomical element. For example, the system may monitor the pose of a vertebra during a spinal surgery or surgical procedure based on readings generated by the independent sensor. Any change in pose of the vertebra (e.g., movement of the vertebra during a drilling procedure) may be reflected in one or more different measurements produced by the independent sensor. In some embodiments, the measurements generated by the independent sensor may be passed to a comparison algorithm (e.g., a comparison algorithm 122), which may compare the newly generated measurements of the independent sensor with previously generated measurements (e.g., measurements taken by the independent sensor before the change in pose) to determine if a change in pose has occurred. For example, a rotation of the vertebra in an XY-plane may be reflected in a changed gravity vector direction reading from the independent sensor. The comparison algorithm may compare the gravity vector measurements of the new measurements with the previous measurements and determine that the vertebra has rotated (and subsequently changed in pose).

In some embodiments, the change in pose may be determined based on both the first set of data and the second set of data. For instance, the comparison algorithm may utilize measurements generated by both the independent sensor and the EM sensor to determine a change in pose of the object. For example, the comparison algorithm may indicate to the system that the object has changed pose when the EM sensor and/or the independent sensor produce measurements that are different from previous measurements (e.g., the EM sensor attached to the object produces EM field measurements that have different vector values than previous measurements, indicating that the tracked object has changed position).

The method 400 also comprises identifying one or more measurements in the first set of data consistent with the determined change of pose (step 420). In some embodiments, the first set of data (which is generated by the EM sensor) may comprise inaccurate measurements (e.g., EM field measurements that have been distorted by the presence of metal in the EM field) as well as accurate measurements (e.g., EM field measurements that have not been distorted by metal or that have not been sufficiently distorted by the metal). The identifying of the one or more measurements may comprise comparing the first set of data (which are generated by the EM sensor) with the one or more datasets generated by the independent sensor to identify one or more measurements from the EM sensor that agree, match, or otherwise accurately reflect the pose of the surgical tool, anatomical element, or other component measured by the independent sensor. For example, the EM sensor may have multiple degrees of freedom (e.g., six degrees of freedom), with one or more measurements associated with each degree of freedom. The comparison algorithm may compare the data generated by the independent sensor (which may have similar degrees of freedom) with the data generated by the EM sensor and identify the measurements of the EM sensor that are inaccurate. In some embodiments, the measurements that are inaccurate may be tagged (e.g., using metadata) or otherwise distinguished from the accurate measurements. For instance, the independent sensor may measure or otherwise capture a gravity vector, which may indicate that a vertebra in a first pose. The EM sensor may have three independent readings (i.e., one reading for each dimension of space) of the EM field near the vertebra, two of which agree, match, or otherwise correctly reflect the first pose of the vertebra, and one of which does not indicate that the vertebra is in the first pose. In this case, the one reading incorrectly indicating the pose of the vertebra may be tagged (e.g., using metadata) as an inaccurate measurement. In some embodiments, the EM sensor may comprise a plurality of EM sensors. In such embodiments, the above identifying step may be performed with each EM sensor, with inaccurate measurements from each EM sensor being tagged as inaccurate. In some embodiments, measurements associated with the worst EM sensor (e.g., the EM sensor from the plurality of EM sensors with the greatest number of inaccurate measurements, the EM sensor from the plurality of EM sensors with the largest inaccuracy, etc.) may be tagged as inaccurate.

The method 400 also comprises determining a second pose of the object using the one or more measurements in the first set of data (step 424). The one or more measurements may be or comprise the measurements from the step 420 that agree, match, or otherwise accurately depict the pose of a component of the system as verified by data obtained from the independent sensor. The one or more measurements may be used by the pose algorithm to determine the second pose. In some embodiments, the inaccurate measurements (i.e., measurements determined by step 424 to be inaccurate) may be omitted from use in determining the second pose. In other words, measurements from the EM sensor that do not agree with the measurements provided by the independent sensor may be removed or omitted from being used when determining the second pose. In some embodiments, the pose algorithm may receive but omit from use the inaccurate measurements (e.g., measurements tagged as inaccurate measurements by the system and/or components thereof). In some embodiments, the second pose may be sent to one or more components of the system (e.g., a navigation system such as a navigation system 118) to update a pose associated with the object.

In some embodiments, the second pose may additionally or alternatively be determined using the second set of data. For instance, the pose algorithm may utilize one or more measurements from the first set of data and/or the second set of data that agree, match, or otherwise accurately depict the pose of the component of the system by data obtained from the independent sensor and/or the EM sensor.

The present disclosure encompasses embodiments of the method 400 that comprise more or fewer steps than those described above, and/or one or more steps that are different than the steps described above.

As noted above, the present disclosure encompasses methods with fewer than all of the steps identified in Figs. 3 and 4 (and the corresponding description of the methods 300 and 400), as well as methods that include additional steps beyond those identified in Figs. 3 and 4 (and the corresponding description of the methods 300 and 400). The present disclosure also encompasses methods that comprise one or more steps from one method described herein, and one or more steps from another method described herein. Any correlation described herein may be or comprise a registration or any other correlation.

## Claims

1. A system (100) comprising:
an electromagnetic, EM, navigation system (118) comprising:
an EM field generator (112) that generates one or more EM fields (115); and
at least one EM sensor (113) affixed to a tracked object,
wherein the EM navigation system (118) determines a pose of the tracked object when the tracked object is within the one or more EM fields (115); and
an independent sensor (136) positioned a first distance relative to the tracked object and that provides a reading indicative of a change in pose of the tracked object,
wherein the reading provided by the independent sensor (136) is useful for determining an accuracy of the pose of the tracked object as determined by the EM navigation system (118),
**characterized in that**
the at least one EM sensor (113) comprises one or more independent sensing coils, and wherein the one or more independent sensing coils measure components of the one or more EM fields (115), and
the determining of the pose by the EM navigation system (118) comprises removing data associated with a first sensing coil of the one or more independent sensing coils based on the information generated by the independent sensor (136).

2. The system (100) of claim 1, wherein the independent sensor (136) comprises an inertial sensor, optionally, wherein the inertial sensor comprises a microelectromechanical sensor (MEMS).

3. The system (100) of claim 1, wherein the independent sensor (136) comprises at least one of an accelerometer and a gyroscope.

4. The system (100) of claim 1, wherein the independent sensor (136) is affixed to the at least one EM sensor (113).

5. The system (100) of claim 1, wherein the independent sensor (136) is affixed to the tracked object, and wherein the independent sensor (136) is substantially smaller than the tracked object.

6. The system (100) of claim 1, wherein the one or more independent sensing coils comprises three orthogonal independent sensing coils.

7. The system (100) of claim 1, wherein the one or more independent sensing coils communicate with a processor (104).

8. The system (100) of claim 1, wherein the one or more independent sensing coils comprises three independent sensing coils, and wherein a measurement associated with at least one independent sensing coil is discarded or replaced by the reading of the independent sensor (136).

9. The system (100) of claim 1, wherein the one or more independent sensing coils comprises three independent sensing coils, and wherein a measurement associated with each of the three independent sensing coils is discarded or replaced by the reading of the independent sensor (136).

10. The system (100) of claim 7, wherein the one or more independent sensing coils comprises one sensing coil, wherein a measurement the one sensing coil indicates a change in direction of the one or more EM fields (115) from a first direction to a second direction, wherein the reading of the independent sensor (136) indicates that the tracked object has not changed position, and wherein the processor (104) disregards the measurement of the one sensing coil in determining the pose of the tracked object.

11. The system (100) of claim 1, wherein the tracked object is a surgical tool, an anatomical element, a surgical robot, or a robotic arm.

12. The system (100) of claim 1, wherein the at least one EM sensor (113) comprises two or more sensing coils, and wherein the two or more sensing coils are at least one of colinear or non-orthogonal.

## Patentansprüche

1. System (100), umfassend:
ein elektromagnetisches Navigationssystem (EM-Navigationssystem) (118), umfassend:
einen EM-Feldgenerator (112), der ein oder mehrere EM-Felder (115) erzeugt; und
mindestens einen EM-Sensor (113), der an einem verfolgten Objekt befestigt ist,
wobei das EM-Navigationssystem (118) eine Stellung des verfolgten Objekts bestimmt, wenn sich das verfolgte Objekt innerhalb des einen oder der mehreren EM-Felder (115) befindet; und
einen unabhängigen Sensor (136), der in einem ersten Abstand relativ zu dem verfolgten Objekt positioniert ist und der einen Messwert bereitstellt, der eine Änderung der Stellung des verfolgten Objekts anzeigt,
wobei der von dem unabhängigen Sensor (136) bereitgestellte Messwert zur Bestimmung einer Genauigkeit der Stellung des verfolgten Objekts, wie durch das EM-Navigationssystem (118) bestimmt, nützlich ist,
**dadurch gekennzeichnet, dass**
der mindestens eine EM-Sensor (113) eine oder mehrere unabhängige Abtastspulen umfasst, und wobei die eine oder die mehreren unabhängigen Abtastspulen Komponenten des einen oder der mehreren EM-Felder (115) messen, und
das Bestimmen der Stellung durch das EM-Navigationssystem (118) das Entfernen von Daten, die mit einer ersten Abtastspule der einen oder mehreren unabhängigen Abtastspulen verbunden sind, basierend auf den durch den unabhängigen Sensor (136) erzeugten Informationen umfasst.

2. System (100) nach Anspruch 1, wobei der unabhängige Sensor (136) einen Trägheitssensor umfasst, wobei optional der Trägheitssensor einen mikroelektromechanischen Sensor (MEMS) umfasst.

3. System (100) nach Anspruch 1, wobei der unabhängige Sensor (136) mindestens einen von einem Beschleunigungsmesser und einem Gyroskop umfasst.

4. System (100) nach Anspruch 1, wobei der unabhängige Sensor (136) an dem mindestens einen EM-Sensor (113) befestigt ist.

5. System (100) nach Anspruch 1, wobei der unabhängige Sensor (136) an dem verfolgten Objekt befestigt ist und wobei der unabhängige Sensor (136) im Wesentlichen kleiner als das verfolgte Objekt ist.

6. System (100) nach Anspruch 1, wobei die eine oder die mehreren unabhängigen Abtastspulen drei orthogonale unabhängige Abtastspulen umfasst.

7. System (100) nach Anspruch 1, wobei die eine oder die mehreren unabhängigen Abtastspulen mit einem Prozessor (104) kommunizieren.

8. System (100) nach Anspruch 1, wobei die eine oder die mehreren unabhängigen Abtastspulen drei unabhängige Abtastspulen umfassen und wobei eine Messung, die mit mindestens einer unabhängigen Abtastspule verbunden ist, verworfen oder durch den Messwert des unabhängigen Sensors (136) ersetzt wird.

9. System (100) nach Anspruch 1, wobei die eine oder die mehreren unabhängigen Abtastspulen drei unabhängige Abtastspulen umfassen und wobei eine Messung, die mit jeder der drei unabhängigen Abtastspulen verbunden ist, verworfen oder durch den Messwert des unabhängigen Sensors (136) ersetzt wird.

10. System (100) nach Anspruch 7, wobei die eine oder die mehreren unabhängigen Abtastspulen eine Abtastspule umfassen, wobei eine Messung der einen Abtastspule eine Richtungsänderung des einen oder der mehreren EM-Felder (115) von einer ersten Richtung zu einer zweiten Richtung anzeigt, wobei der Messwert des unabhängigen Sensors (136) anzeigt, dass das verfolgte Objekt seine Position nicht geändert hat, und wobei der Prozessor (104) die Messung der einen Abtastspule beim Bestimmen der Stellung des verfolgten Objekts außer Acht lässt.

11. System (100) nach Anspruch 1, wobei das verfolgte Objekt ein chirurgisches Instrument, ein anatomisches Element, ein chirurgischer Roboter oder ein Roboterarm ist.

12. System (100) nach Anspruch 1, wobei der mindestens eine EM-Sensor (113) zwei oder mehr Abtastspulen umfasst und wobei die zwei oder mehr Abtastspulen mindestens eines von kollinear oder nichtorthogonal sind.

## Revendications

1. Système (100) comprenant :
un système de navigation électromagnétique, EM (118) comprenant :
un générateur de champs EM (112) qui génère un ou plusieurs champs EM (115) ; et
au moins un capteur EM (113) fixé à un objet suivi,
dans lequel le système de navigation EM (118) détermine une position de l'objet suivi lorsque l'objet suivi se trouve dans le ou les champs EM (115) ; et
un capteur indépendant (136) positionné à une première distance par rapport à l'objet suivi et qui fournit une lecture indiquant un changement de position de l'objet suivi,
dans lequel la lecture fournie par le capteur indépendant (136) est utile pour déterminer une précision de la position de l'objet suivi telle que déterminée par le système de navigation EM (118),
**caractérisé en ce que**
l'au moins un capteur EM (113) comprend une ou plusieurs bobines de détection indépendantes, et dans lequel la ou les bobines de détection indépendantes mesurent des composants du ou des champs EM (115), et
la détermination de la position par le système de navigation EM (118) comprend la suppression de données associées à une première bobine de détection de la ou des bobines de détection indépendantes en fonction des informations générées par le capteur indépendant (136).

2. Système (100) selon la revendication 1, dans lequel le capteur indépendant (136) comprend un capteur inertiel, éventuellement, dans lequel le capteur inertiel comprend un capteur microélectromécanique (MEMS).

3. Système (100) selon la revendication 1, dans lequel le capteur indépendant (136) comprend au moins l'un parmi un accéléromètre et un gyroscope.

4. Système (100) selon la revendication 1, dans lequel le capteur indépendant (136) est fixé à l'au moins un capteur EM (113).

5. Système (100) selon la revendication 1, dans lequel le capteur indépendant (136) est fixé à l'objet suivi, et dans lequel le capteur indépendant (136) est sensiblement plus petit que l'objet suivi.

6. Système (100) selon la revendication 1, dans lequel la ou les bobines de détection indépendantes comprennent trois bobines de détection indépendantes orthogonales.

7. Système (100) selon la revendication 1, dans lequel la ou les bobines de détection indépendantes communiquent avec un processeur (104).

8. Système (100) selon la revendication 1, dans lequel la ou les bobines de détection indépendantes comprennent trois bobines de détection indépendantes, et dans lequel une mesure associée à au moins une bobine de détection indépendante est écartée ou remplacée par la lecture du capteur indépendant (136).

9. Système (100) selon la revendication 1, dans lequel la ou les bobines de détection indépendantes comprennent trois bobines de détection indépendantes, et dans lequel une mesure associée à chacune des trois bobines de détection indépendantes est écartée ou remplacée par la lecture du capteur indépendant (136).

10. Système (100) selon la revendication 7, dans lequel la ou les bobines de détection indépendantes comprennent une bobine de détection, dans lequel une mesure de la bobine de détection indique un changement de direction du ou des champs EM (115) d'une première direction à une seconde direction, dans lequel la lecture du capteur indépendant (136) indique que l'objet suivi n'a pas changé de position, et dans lequel le processeur (104) ne tient pas compte de la mesure de la bobine de détection pour déterminer la position de l'objet suivi.

11. Système (100) selon la revendication 1, dans lequel l'objet suivi est un outil chirurgical, un élément anatomique, un robot chirurgical ou un bras robotique.

12. Système (100) selon la revendication 1, dans lequel l'au moins un capteur EM (113) comprend deux ou plusieurs bobines de détection, et dans lequel les deux ou plusieurs bobines de détection sont au moins l'une parmi colinéaire ou non orthogonale.
